Europäisches Patentamt

(19) European Patent Office

Office européen des brevets

(11) Publication number : **0 459 747 A1**

# (12) EUROPEAN PATENT APPLICATION

(21) Application number : **91304796.5**

(22) Date of filing : **28.05.91**

(51) Int. Cl.⁵ : **C12N 15/16, C07K 7/10, A61K 37/43**

The applicant has subsequently filed a sequence listing and declared, that it includes no new matter.

(30) Priority : **29.05.90 US 530150**

(43) Date of publication of application :
**04.12.91 Bulletin 91/49**

(84) Designated Contracting States :
**AT BE CH DE DK ES FR GB GR IT LI LU NL SE**

(71) Applicant : **ELI LILLY AND COMPANY**
**Lilly Corporate Center**
**Indianapolis Indiana 46285 (US)**

(72) Inventor : **Chan, Ging**
**6205 Newberry Road, Apartment 1408**
**Indianapolis, Indiana 46256 (US)**
Inventor : **Heiman, Mark Louis**
**5740 Susan Drive East**
**Indianapolis, Indiana 46250 (US)**
Inventor : **Hsiung, Hansen Maxwell**
**4760 Cole Porter Lane**
**Indianapolis, Indiana 46032 (US)**

(74) Representative : **Hudson, Christopher Mark et al**
**Erl Wood Manor**
**Windlesham Surrey GU20 6PH (GB)**

(54) **Precursor forms of porcine growth hormone releasing factor and related DNA compounds.**

(57)  The present invention provides polypeptide compounds which are two forms of the porcine growth hormone releasing factor (pGRF) precursor and the pGRF leader peptide, and a new form of mature pGRF. The invention also provides DNA compounds encoding these polypeptide compounds and mature pGRF, pharmaceutical compositions and methods for the inducement of growth hormone release.

EP 0 459 747 A1

Growth hormone releasing factor (GRF) is useful to stimulate the production of growth hormone in humans and animals. Porcine GRF (pGRF) is of particular interest for veterinary use. The amino acid sequence of mature pGRF has been elucidated. Bohlen et al., Biochem. Biophys. Res. Commun. 116:726 (1983). However, the DNA sequence encoding pGRF in nature was unknown until the present invention. Provided herein are previously unknown polypeptide compounds which comprise two forms of the pGRF precursor and the pGRF peptide. Also included are DNA compounds encoding polypeptide compounds of the invention and mature pGRF.

The present invention provides DNA compounds that comprise DNA sequences encoding porcine growth hormone releasing factor, two forms of the pGRF precursor, and the pGRF leader peptide. Also provided are polypeptide compounds including two forms of the pGRF precursor in addition to the pGRF leader peptide linked to the two pGRF precursor forms.

The polypeptide compounds of the invention are of the formula

```
A-Tyr-Ala-Asp-Ala-Ile-Phe-Thr-Asn-B-Tyr-Arg-Lys-Val-
Leu-Gly-Gln-Leu-Ser-Ala-Arg-Lys-Leu-Leu-Gln-Asp-Ile-
Met-Ser-Arg-Gln-Gln-Gly-Glu-Arg-Asn-Gln-Glu-Gln-Gly-
Ala-Arg-Val-Arg-Leu-Gly-Arg-Gln-Val-Asp-Ser-Met-Trp-
Ala-Asp-Gln-Lys-Gln-Met-Ala-Leu-Glu-Ser-Ile-Leu-Ala-
Thr-Leu-Leu-Gln-Glu-His-Arg-Asn-Ser-Gln-Gly-OH;
```

```
wherein A is hydrogen or H-Met-Leu-Leu-Trp-Val-Phe-Phe-
Leu-Val-Thr-Leu-Thr-Leu-Ser-Ser-Gly-Ser-Leu-Ser-Ser-Leu-
Pro-Ser-Gln-Pro-Leu-Arg-Met-Pro-Arg;  B is Asn or Ser;
```

and the pharmaceutically acceptable acid or carboxylic acid addition salts thereof. When A is hydrogen the compound represents the pGRF precursor. Cloning of the pGRF coding sequence by the present inventors surprisingly has revealed that there are at least two alleles of the pGRF gene. The alleles differ by the codon encoding the amino acid at position 9 of mature pGRF. One allele encodes an asparagine at that position; the other allele encodes a serine. The differences in the alleles is represented by the variable B.

An additional polypeptide compound of the invention is the $Asn^9$ form of mature pGRF which is

```
H-Tyr-Ala-Asp-Ala-Ile-Phe-Thr-Asn-Asn-Tyr-Arg-Lys-Val-
Leu-Gly-Gln-Leu-Ser-Ala-Arg-Lys-Leu-Leu-Gln-Asp-Ile-
Met-Ser-Arg-Gln-Gln-Gly-Glu-Arg-Asn-Gln-Glu-Gln-Gly-
Ala-Arg-Val-Arg-Leu-A;  wherein A is OH or NH_2 .
```

The DNA compounds of the invention comprise DNA sequences encoding two forms of mature pGRF, two forms of the pGRF precursor, and the pGRF leader peptide.

A preferred DNA sequence encoding the $Asn^9$ form of mature pGRF is

```
TATGCAGATGCCATCTTCACCAACAACTACCGGAAGGTGCTGGGCCA
---------+---------+---------+---------+-------
ATACGTCTACGGTAGAAGTGGTTGTTGATGGCCTTCCACGACCCGGT

GCTCTCTGCCCGAAAGCTCCTCCAGGACATCATGAGCAGGCAGCAGG
--+---------+---------+---------+---------+----
CGAGAGACGGGCTTTCGAGGAGGTCCTGTAGTACTCGTCCGTCGTCC

GGGAGAGAAACCAGGAGCAAGGA
-----+---------+-------
CCCTCTCTTTGGTCCTCGTTCCT    .
```

The other form of mature pGRF, differing by the serine at position 9, is preferably encoded by the DNA sequence

```
TATGCAGATGCCATCTTCACCAACAGCTACCGGAAGGTGCTGGGCCA
---------+---------+---------+---------+-------
ATACGTCTACGGTAGAAGTGGTTGACGATGGCCTTCCACGACCCGGT

GCTCTCTGCCCGAAAGCTCCTCCAGGACATCATGAGCAGGCAGCAGG
--+---------+---------+---------+---------+----
CGAGAGACGGGCTTTCGAGGAGGTCCTGTAGTACTCGTCCGTCGTCC

GGGAGAGAAACCAGGAGCAAGGA
-----+---------+-------
CCCTCTCTTTGGTCCTCGTTCCT    .
```

A preferred DNA sequence encoding the Asn[9] form of the pGRF precursor is

```
TATGCAGATGCCATCTTCACCAACAACTACCGGAAGGTGCTGGGCCA
---------+---------+---------+---------+-------
ATACGTCTACGGTAGAAGTGGTTGTTGATGGCCTTCCACGACCCGGT

GCTCTCTGCCCGAAAGCTCCTCCAGGACATCATGAGCAGGCAGCAGG
--+---------+---------+---------+---------+----
CGAGAGACGGGCTTTCGAGGAGGTCCTGTAGTACTCGTCCGTCGTCC

GGGAGAGAAACCAGGAGCAAGGAGCAAGGGTACGGCTTGGCCGTCAG
-----+---------+---------+---------+---------+-
CCCTCTCTTTGGTCCTCGTTCCTCGTTCCCATGCCGAACCGGCAGTC

GTGGACAGCATGTGGGCAGACCAAAAGCAGATGGCATTGGAGAGCAT
--------+---------+---------+---------+--------
CACCTGTCGTACACCCGTCTGGTTTTCGTCTACCGTAACCTCTCGTA

CCTGGCGACCCTGCTGCAGGAGCACAGGAATTCCCAAGGATGA
---------+---------+---------+---------+---
GGACCGCTGGGACGACGTCCTCGTGTCCTTAAGGGTTCCTACT    .
```

The Ser[9] form of the precursor is preferably encoded by the DNA sequence

```
TATGCAGATGCCATCTTCACCAACAGCTACCGGAAGGTGCTGGGCCA
---------+---------+---------+---------+-------
ATACGTCTACGGTAGAAGTGGTTGTCGATGGCCTTCCACGACCCGGT

GCTCTCTGCCCGAAAGCTCCTCCAGGACATCATGAGCAGGCAGCAGG
--+---------+---------+---------+---------+----
CGAGAGACGGGCTTTCGAGGAGGTCCTGTAGTACTCGTCCGTCGTCC

GGGAGAGAAACCAGGAGCAAGGAGCAAGGGTACGGCTTGGCCGTCAG
-----+---------+---------+---------+---------+-
CCCTCTCTTTGGTCCTCGTTCCTCGTTCCCATGCCGAACCGGCAGTC

GTGGACAGCATGTGGGCAGACCAAAAGCAGATGGCATTGGAGAGCAT
--------+---------+---------+---------+--------
CACCTGTCGTACACCCGTCTGGTTTTCGTCTACCGTAACCTCTCGTA

CCTGGCGACCCTGCTGCAGGAGCACAGGAATTCCCAAGGATGA
---------+---------+---------+---------+---
GGACCGCTGGGACGACGTCCTCGTGTCCTTAAGGGTTCCTACT    .
```

A preferred DNA sequence encoding the Asn[9] form of the pGRF precursor preceded by its leader peptide sequence is

```
                    ATGCTGCTCTGGGTGTTCTTCCTCGTCACCCT
                    ---------+---------+---------+--
                    TACGACGAGACCCACAAGAAGGAGCAGTGGGA

CACCCTCAGCAGCGGCTCCCTCAGCTCCCTGCCCTCCCAGCCCCTCAGGATGCCGC
-------+---------+---------+---------+---------+------+--
GTGGGAGTCGTCGCCGAGGGAGTCGAGGGACGGGAGGGTCGGGGAGTCCTACGGCG

GGTATGCAGATGCCATCTTCACCAACAACTACCGGAAGGTGCTGGGCCAGCTCTCT
-------+---------+---------+---------+---------+--------
CCATACGTCTACGGTAGAAGTGGTTGTTGATGGCCTTCCACGACCCGGTCGAGAGA

GCCCGAAAGCTCCTCCAGGACATCATGAGCAGGCAGCAGGGGGAGAGAAACCAGGA
-+---------+---------+---------+---------+---------+----
CGGGCTTTCGAGGAGGTCCTGTAGTACTCGTCCGTCGTCCCCCCTCTCTTTGGTCCT

GCAAGGAGCAAGGGTACGGCTTGGCCGTCAGGTGGACAGCATGTGGGCAGACCAAA
-----+---------+---------+---------+---------+---------+
CGTTCCTCGTTCCCATGCCGAACCGGCAGTCCACCTGTCGTACACCCGTCTGGTTT

AGCAGATGGCATTGGAGAGCATCCTGGCGACCCTGCTGCAGGAGCACAGGAATTCC
---------+---------+---------+---------+---------+------
TCGTCTACCGTAACCTCTCGTAGGACCGCTGGGACGACGTCCTCGTGTCCTTAAGG

CAAGGATGA
---+-----
GTTCCTACT    .
```

The Ser[9] form of the pGRF precursor and its leader sequence is preferably encoded by

```
ATGCTGCTCTGGGTGTTCTTCCTCGTCACCCT
---------+---------+---------+--
TACGACGAGACCCACAAGAAGGAGCAGTGGGA

CACCCTCAGCAGCGGCTCCCTCAGCTCCCTGCCCTCCCAGCCCCTCAGGATGCCGC
-------+---------+---------+---------+---------+--------
GTGGGAGTCGTCGCCGAGGGAGTCGAGGGACGGGAGGGTCGGGGAGTCCTACGGCG

GGTATGCAGATGCCATCTTCACCAGCAACTACCGGAAGGTGCTGGGCCAGCTCTCT
--+---------+---------+---------+---------+---------+--
CCATACGTCTACGGTAGAAGTGGTCGTTGATGGCCTTCCACGACCCGGTCGAGAGA

GCCCGAAAGCTCCTCCAGGACATCATGAGCAGGCAGCAGGGGGAGAGAAACCAGGA
-------+---------+---------+---------+---------+--------
CGGGCTTTCGAGGAGGTCCTGTAGTACTCGTCCGTCGTCCCCCTCTCTTTGGTCCT

GCAAGGAGCAAGGGTACGGCTTGGCCGTCAGGTGGACAGCATGTGGGCAGACCAAA
--+---------+---------+---------+---------+---------+---
CGTTCCTCGTTCCCATGCCGAACCGGCAGTCCACCTGTCGTACACCCGTCTGGTTT

AGCAGATGGCATTGGAGAGCATCCTGGCGACCCTGCTGCAGGAGCACAGGAATTCC
------+---------+---------+---------+---------+---------
TCGTCTACCGTAACCTCTCGTAGGACCGCTGGGACGACGTCCTCGTGTCCTTAAGG

CAAGGATGA
+--------
GTTCCTACT    .
```

Also provided by the present invention are pharmaceutical compositions where the active ingredient is a polypeptide compound comprising either the Asn$^9$ or Ser$^9$ form of the precursor. Finally, the invention provides methods for inducing growth hormone release, which comprises administering polypeptide compounds comprising either pGRF precursor form. The compositions and methods may be useful for inducing growth hormone release in a variety of animals, including humans, pigs, cattle, sheep, chickens and fish. The preferred uses of the compositions and methods are in pigs.

The following section provides a more detailed description of the present invention. For purposes of clarity and as an aid in the understanding of the invention, as disclosed and claimed herein, the following terms and abbreviations are defined below.

GRF - a polypeptide with growth hormone releasing factor activity.

GRF(1-29)NH$_2$ - the minimal portion of GRF that is required for full potency.

H - the hydrogen atom present on the amino terminal ends of polypeptides and proteins.

High-Level Expression in E. coli - the production of a GRF analog encoded by a cloned gene at levels such that the gene product is detectable by Coomassie Blue staining.

Isolated DNA Sequence - any DNA sequence, however constructed or synthesized, which is locationally distinct from its natural location in genomic DNA. The definition includes the isolated DNA sequence in all its forms other than the natural state. For example, the DNA sequence may be inserted into a plasmid or phage vector or inserted into the genome of the organism from which it came or any other organism to increase the gene dosage.

```
    Mature pGRF - pGRF (1-44); H-Tyr-Ala-Asp-Ala-
Ile-Phe-Thr-Asn-B-Tyr-Arg-Lys-Val-Leu-Gly-Gln-Leu-Ser-
Ala-Arg-Lys-Leu-Leu-Gln-Asp-Ile-Met-Ser-Arg-Gln-Gln-
Gly-Glu-Arg-Asn-Gln-Glu-Gln-Gly-Ala-Arg-Val-Arg-Leu-OH,
where B is Asn or Ser.
    pGRF precursor - pGRF (1-76);
    H-Tyr-Ala-Asp-Ala-Ile-Phe-Thr-Asn-B-Tyr-Arg-Lys-Val-
    Leu-Gly-Gln-Leu-Ser-Ala-Arg-Lys-Leu-Leu-Gln-Asp-Ile-
    Met-Ser-Arg-Gln-Gln-Gly-Glu-Arg-Asn-Gln-Glu-Gln-Gly-
    Ala-Arg-Val-Arg-Leu-Gly-Arg-Gln-Val-Asp-Ser-Met-Trp-
    Ala-Asp-Gln-Lys-Gln-Met-Ala-Leu-Glu-Ser-Ile-Leu-Ala-
    Thr-Leu-Leu-Gln-Glu-His-Arg-Asn-Ser-Gln-Gly-OH;
```

wherein B is Asn or Ser.

Standard three letter amino acid abbreviations are used throughout the document.

The present invention provides isolated DNA compounds which comprise DNA sequences encoding two forms of mature growth hormone releasing factor, two forms of the pGRF precursor, and the pGRF leader peptide linked to a pGRF precursor. The invention also provides polypeptide compounds.

Two of the polypeptide compounds of the invention are encoded by two different alleles of the pGRF gene. Although the amino acid structure of a form of mature pGRF has been previously elucidated (Bohlen et al., Biochem. Biophys. Res. Commun. 116: 726 (1983)), the present invention provides two forms of the heretofore unknown pGRF precursor. The precursor compounds are represented by the formula

```
    H-Tyr-Ala-Asp-Ala-Ile-Phe-Thr-Asn-B-Tyr-Arg-Lys-Val-
    Leu-Gly-Gln-Leu-Ser-Ala-Arg-Lys-Leu-Leu-Gln-Asp-Ile-
    Met-Ser-Arg-Gln-Gln-Gly-Glu-Arg-Asn-Gln-Glu-Gln-Gly-
    Ala-Arg-Val-Arg-Leu-Gly-Arg-Gln-Val-Asp-Ser-Met-Trp-
    Ala-Asp-Gln-Lys-Gln-Met-Ala-Leu-Glu-Ser-Ile-Leu-Ala-
    Thr-Leu-Leu-Gln-Glu-His-Arg-Asn-Ser-Gln-Gly-OH
```

wherein B is Asn or Ser; and the pharmaceutically acceptable acid or carboxylic acid addition salt thereof. The pGRF precursor is especially useful because it fills a need in the art for a compound with pGRF activity that can be produced at high levels in E. coli. The compounds are of sufficient size that the serious degradation problem encountered with expression of mature GRF in E. coli is escaped. See e.g., Kirschner et al., J. Biotech. 12: 247, 248 (1989).

Specific pGRF precursor compounds are

```
    H-Tyr-Ala-Asp-Ala-Ile-Phe-Thr-Asn-Asn-Tyr-Arg-Lys-Val-
    Leu-Gly-Gln-Leu-Ser-Ala-Arg-Lys-Leu-Leu-Gln-Asp-Ile-
    Met-Ser-Arg-Gln-Gln-Gly-Glu-Arg-Asn-Gln-Glu-Gln-Gly-
    Ala-Arg-Val-Arg-Leu-Gly-Arg-Gln-Val-Asp-Ser-Met-Trp-
    Ala-Asp-Gln-Lys-Gln-Met-Ala-Leu-Glu-Ser-Ile-Leu-Ala-
    Thr-Leu-Leu-Gln-Glu-His-Arg-Asn-Ser-Gln-Gly-OH;  and
```

the pharmaceutically acceptable acid or carboxylic acid addition salts thereof
and

```
H-Tyr-Ala-Asp-Ala-Ile-Phe-Thr-Asn-Ser-Tyr-Arg-Lys-Val-
   Leu-Gly-Gln-Leu-Ser-Ala-Arg-Lys-Leu-Leu-Gln-Asp-Ile-
   Met-Ser-Arg-Gln-Gln-Gly-Glu-Arg-Asn-Gln-Glu-Gln-Gly-
   Ala-Arg-Val-Arg-Leu-Gly-Arg-Gln-Val-Asp-Ser-Met-Trp-
   Ala-Asp-Gln-Lys-Gln-Met-Ala-Leu-Glu-Ser-Ile-Leu-Ala-
   Thr-Leu-Leu-Gln-Glu-His-Arg-Asn-Ser-Gln-Gly-OH;  and
```

the pharmaceutically acceptable acid or carboxylic acid addition salts thereof.

Other polypeptide compounds of the invention are the pGRF precursor forms with the pGRF leader peptide attached to the precursors' amino terminii. These compounds are represented by the formula

```
H-Met-Leu-Leu-Trp-Val-Phe-Phe-Leu-Val-Thr-Leu-Thr-Leu-
   Ser-Ser-Gly-Ser-Leu-Ser-Ser-Leu-Pro-Ser-Gln-Pro-Leu-
   Arg-Met-Pro-Arg-Tyr-Ala-Asp-Ala-Ile-Phe-Thr-Asn-B-
   Tyr-Arg-Lys-Val-Leu-Gly-Gln-Leu-Ser-Ala-Arg-Lys-Leu-
   Leu-Gln-Asp-Ile-Met-Ser-Arg-Gln-Gln-Gly-Glu-Arg-Asn-
   Gln-Glu-Gln-Gly-Ala-Arg-Val-Arg-Leu-Gly-Arg-Gln-Val-
   Asp-Ser-Met-Trp-Ala-Asp-Gln-Lys-Gln-Met-Ala-Leu-Glu-
   Ser-Ile-Leu-Ala-Thr-Leu-Leu-Gln-Glu-His-Arg-Asn-Ser-
   Gln-Gly-OH;
```

wherein B is Asn or Ser.

These compounds are particularly useful when they are produced in mammalian cells. The leader peptide causes secretion of the polypeptide product; cleavage of the leader peptide occurs as the product is secreted. Specific pGRF precursor compounds comprising the pGRF leader peptide are

```
H-Met-Leu-Leu-Trp-Val-Phe-Phe-Leu-Val-Thr-Leu-Thr-Leu-
   Ser-Ser-Gly-Ser-Leu-Ser-Ser-Leu-Pro-Ser-Gln-Pro-Leu-
   Arg-Met-Pro-Arg-Tyr-Ala-Asp-Ala-Ile-Phe-Thr-Asn-Asn-
   Tyr-Arg-Lys-Val-Leu-Gly-Gln-Leu-Ser-Ala-Arg-Lys-Leu-
   Leu-Gln-Asp-Ile-Met-Ser-Arg-Gln-Gln-Gly-Glu-Arg-Asn-
   Gln-Glu-Gln-Gly-Ala-Arg-Val-Arg-Leu-Gly-Arg-Gln-Val-
   Asp-Ser-Met-Trp-Ala-Asp-Gln-Lys-Gln-Met-Ala-Leu-Glu-
   Ser-Ile-Leu-Ala-Thr-Leu-Leu-Gln-Glu-His-Arg-Asn-Ser-
   Gln-Gly-OH
```

and

```
H-Met-Leu-Leu-Trp-Val-Phe-Phe-Leu-Val-Thr-Leu-Thr-Leu-
   Ser-Ser-Gly-Ser-Leu-Ser-Ser-Leu-Pro-Ser-Gln-Pro-Leu-
   Arg-Met-Pro-Arg-Tyr-Ala-Asp-Ala-Ile-Phe-Thr-Asn-Asn-
   Tyr-Arg-Lys-Val-Leu-Gly-Gln-Leu-Ser-Ala-Arg-Lys-Leu-
   Leu-Gln-Asp-Ile-Met-Ser-Arg-Gln-Gln-Gly-Glu-Arg-Asn-
   Gln-Glu-Gln-Gly-Ala-Arg-Val-Arg-Leu-Gly-Arg-Gln-Val-
   Asp-Ser-Met-Trp-Ala-Asp-Gln-Lys-Gln-Met-Ala-Leu-Glu-
   Ser-Ile-Leu-Ala-Thr-Leu-Leu-Gln-Glu-His-Arg-Asn-Ser-
   Gln-Gly-OH.
```

All of the peptide compounds of the invention can be made by chemical methods well-known in the art, including solid phase peptide synthesis or recombinant methods. Both methods are described in U.S. Patent 4,617,149, herein incorporated by reference. Recombinant methods are preferred if a high yield is desired. A general method for the construction of any desired DNA sequence is provided in Brown et al., Methods in Enzymology 68:109 (1979), herein incorporated by reference.

The DNA sequences can be synthesized using automated DNA synthesizers, such as the ABS (Applied Biosystems, 850 Lincoln Centre Drive, Foster City, CA 94404) 380B DNA synthesizer. The DNA sequences can also be generated by the polymerase chain reaction. See, e.g., U.S. Patents 4,800,159 and 4,683,202 and European Patent Publication No. 0258017, published March 2, 1987.

The amino acid sequences of the polypeptide compounds of the invention can be encoded by a multitude of different DNA sequences because most of the amino acids are encoded by more than one DNA triplet. Because these alternate DNA sequences would encode the same amino acid sequences, the present invention further comprises these alternate sequences.

Skilled artisans recognize that the initiation of translation of polypeptides in E. coli is via a methionine residue. The polypeptides of the invention, other than those that have the pGRF leader peptide at their amino terminii, do not necessarily begin with methionine. Therefore, the compounds may be produced in several ways. After expression of the polypeptide compound, a leader sequence of methionine or methionine and additional amino acids are removed by any of several well-known routes. See, e.g., U.S. Patents 4,745,069 and 4,782,139; Villa et al., Eur. J. Biochem. 171:137 (1988); Geli et al., Gene 80:129 (1989); and European Patent Publication No. 0199018.

Other routes of production are well-known to skilled artisans. For example, the compounds are produced in eucaryotic cells using SV40-derived vectors. Expression in eucaryotic cells is achieved via the pGRF precursor cDNA sequence. Such a method is described in U.S. Patent 4,775,624. Several alternate methods of expression are described in J. Sambrook, E.F. Fritsch & T. Maniatis, Molecular Cloning: A Laboratory Manual 16.3-17.44 (1989). Gene expression in Saccharomyces cerevisiae is detailed in 1 Current Protocols in Molecular Biology (F.M. Ausubel, R.Brent, R.E. Kingston, D.D. Moore, J.G. Seidman, J.A. Smith & K. Struhl 1989).

The DNA sequences of the present invention encode the polypeptide compounds of the present invention and mature pGRF. The amino acid sequence of one form of pGRF is known but the DNA sequence encoding it was unknown until the present invention.

Included in the polypeptide compounds of this invention are their pharmaceutically acceptable non-toxic acid addition salts and their pharmaceutically acceptable non-toxic carboxylic acid salts.

The term "pharmaceutically acceptable non-toxic acid addition salts" encompasses both organic and inorganic acid addition salts including, for example, those prepared from acids such as hydrochloric, hydrofluoric, sulfuric, sulfonic, tartaric, fumaric, hydrobromic, glycolic, citric, maleic, phosphoric, succinic, acetic, nitric, benzoic, ascorbic, p-toluenesulfonic, benzenesulfonic, naphthalenesulfonic, propionic, and the like. Preferably, the acid addition salts are those prepared from hydrochloric acid, acetic acid, or succinic acid. Any of the above salts is prepared by conventional methods.

The term "carboxylic acid salts" includes, for example, amine, ammonium, quaternary ammonium, alkali metal and alkaline earth metal salts such as calcium, magnesium, sodium, potassium, and lithium, and the like.

The present invention also provides a pharmaceutical composition comprising as the active agent a polypeptide compound or a pharmaceutically acceptable acid or carboxylic acid addition salt thereof, wherein said polypeptide compound has the formula

```
H-Tyr-Ala-Asp-Ala-Ile-Phe-Thr-Asn-A-Tyr-Arg-Lys-Val-
Leu-Gly-Gln-Leu-Ser-Ala-Arg-Lys-Leu-Leu-Gln-Asp-Ile-
Met-Ser-Arg-Gln-Gln-Gly-Glu-Arg-Asn-Gln-Glu-Gln-Gly-
Ala-Arg-Val-Arg-Leu-Gly-Arg-Gln-Val-Asp-Ser-Met-Trp-
Ala-Asp-Gln-Lys-Gln-Met-Ala-Leu-Glu-Ser-Ile-Leu-Ala-
Thr-Leu-Leu-Gln-Glu-His-Arg-Asn-Ser-Gln-Gly-OH;
```

wherein A is Asn or Ser; and a pharmaceutically acceptable solid or liquid carrier.

Also provided in the present invention is a method for inducing growth hormone release which comprises administering an effective amount of a polypeptide compound represented by the formula

```
H-Tyr-Ala-Asp-Ala-Ile-Phe-Thr-Asn-A-Tyr-Arg-Lys-Val-
Leu-Gly-Gln-Leu-Ser-Ala-Arg-Lys-Leu-Leu-Gln-Asp-Ile-
Met-Ser-Arg-Gln-Gln-Gly-Glu-Arg-Asn-Gln-Glu-Gln-Gly-
Ala-Arg-Val-Arg-Leu-Gly-Arg-Gln-Val-Asp-Ser-Met-Trp-
Ala-Asp-Gln-Lys-Gln-Met-Ala-Leu-Glu-Ser-Ile-Leu-Ala-
Thr-Leu-Leu-Gln-Glu-His-Arg-Asn-Ser-Gln-Gly-OH
```

wherein A is Asn or Ser; and the pharmaceutically acceptable acid or carboxylic acid addition salts thereof. Doses of the precursor compounds of this invention are administered to the recipient for a period during which stimulation of the release of growth hormone is desired. The weight of the recipient and mode of administration will have an influence upon the size of the dose necessary to induce a particular response. Preferred doses in pigs are estimated to be about 0.3-12 mg/day.

In administering the polypeptide compounds of this invention parenterally, the pharmaceutical forms suitable for injection include sterile aqueous solutions or dispersions and sterile powders for reconstitution into sterile injectable solutions or dispersions. The carrier can be a solvent or dispersing medium containing, for example, water, ethanol, polyol (for example glycerol, propylene glycol, liquid polyethylene glycol, and the like), suitable mixtures thereof, or vegetable oils. Proper fluidity can be maintained, for example, by the use of a coating such as lecithin, by the maintenance of the required particle size in the case of dispersion and by the use of surfactants. Prevention of the action of microorganisms can be ensured by various antibacterial and antifungal agents, for example, parabens, chlorobutanol, phenol, sorbic acid, and the like. In many cases, it will be desirable to include isotonic agents, for example, sugars, sodium chloride, and the like. Prolonged absorption of the injectable pharmaceutical form can be brought about by the use of agents delaying absorption, for example, aluminum monostearate and gelatin.

Sterile injectable solutions can be prepared by incorporating the compounds of this invention in the required amount of the appropriate solvent with various of the other ingredients, as desired. If desired, and for more effective distribution, the compounds can be incorporated into slow release or targeted delivery systems such as polymer matrices, liposomes, and microspheres. The compounds may be delivered via mechanical release devices, osmotic pumps, or any other release device or system which provides continuous or pulsatile delivery. Because the art of the pharmaceutical administration of peptide compounds is in its infancy, it is anticipated that significant advances in this technology will occur. The compounds of the invention will be useful as administered by these new methods.

It is especially advantageous to formulate the compounds of this invention in unit dosage form for ease of administration and uniformity of dosage. Unit dosage form as used herein refers to physically discrete units suited as unitary dosages for the subject to be treated. Each unit contains a predetermined quantity of the compound calculated to produce the desired therapeutic effect in association with the pharmaceutically acceptable carrier. The specific unit dosage form is dictated by and directly dependent upon (a) the unique characteristics of the particular composition and (b) the particular therapeutic effect to be achieved.

The following examples are illustrative of this invention. They are not intended to be limiting upon the scope thereof.

9

Example 1

Expression inE.coliof a pGRF Precursor

The plasmid is constructed and the polypeptide expressed using the procedures of U.S. Patent 4,828,988, herein incorporated by reference. The procedure is modified so that a pGRF precursor coding sequence is substituted for the human GRF coding sequences described in that patent. A suitable pGRF precursor coding sequence is

```
TATGCAGATGCCATCTTCACCAACAACTACCGGAAGGTGCTGGGCCA
---------+---------+---------+---------+-------
ATACGTCTACGGTAGAAGTGGTTGTTGATGGCCTTCCACGACCCGGT

GCTCTCTGCCCGAAAGCTCCTCCAGGACATCATGAGCAGGCAGCAGG
--+---------+---------+---------+---------+----
CGAGAGACGGGCTTTCGAGGAGGTCCTGTAGTACTCGTCCGTCGTCC

GGGAGAGAAACCAGGAGCAAGGAGCAAGGGTACGGCTTGGCCGTCAG
-----+---------+---------+---------+---------+-
CCCTCTCTTTGGTCCTCGTTCCTCGTTCCCATGCCGAACCGGCAGTC

GTGGACAGCATGTGGGCAGACCAAAAGCAGATGGCATTGGAGAGCAT
--------+---------+---------+---------+--------
CACCTGTCGTACACCCGTCTGGTTTTCGTCTACCGTAACCTCTCGTA

CCTGGCGACCCTGCTGCAGGAGCACAGGAATTCCCAAGGATGA
---------+---------+---------+---------+---
GGACCGCTGGGACGACGTCCTCGTGTCCTTAAGGGTTCCTACT    .
```

The DNA sequence encodes the Asn$^9$ form of the pGRF precursor. The Ser$^9$ form may be generated by changing the coding sequence of the ninth amino acid from AAC to AGC.

Example 2

Purification of the pGRF Precursor

A cation exchange column is prepared using S Sepharose® (Pharmacia, 800 Centennial Ave., Piscataway, NJ 08854) resin. The column contains one liter of resin per 50 g of material. The pGRF precursor containing material is added to the column at a flow rate of 0.1 l/cm$^2$/hr and washed with 2 column volumes of 0.1 M sodium chloride in 0.05 N acetic acid-7M urea. The polypeptide is eluted by a linear gradient of 0.25 M to 1.6 M sodium chloride in acetic acid-urea using three column volumes of each with 0.1 column volume fractions collected. The polypeptide-containing fractions are identified by conductivity, O.D.$_{276}$, HPLC and polyacrylamide gel electrophoresis. The fractions are then pooled.

An equal volume of acetic acid-urea solution is added to the pooled fractions. The material is then applied to a column containing S Sepharose® resin in acetic acid-urea sized to accommodate 50 g of protein per liter of resin. The flow rate is 0.02 l/cm$^2$/hr. The pGRF precursor fractions are eluted by a linear gradient of 0.25 M to 1.2 M sodium chloride in acetic acid-urea. Fractions of 0.1 column volume are collected. The fractions are analyzed as before and the pGRF precursor-containing fractions are pooled.

A Sephadex® G-15 (Pharmacia) column is prepared in 0.02 M glycine, pH 2.5 with a column volume five times the volume of the previously pooled fractions. Fractions containing the O.D.$_{276}$ peak are isolated.

A column containing SP20SS resin (Sephabeads, Mitsubishi Chemical, Tokyo) in 10% acetonitrile-0.02 M glycine, pH 2.5, is then prepared. The pooled pGRF precursor-containing solution is made 10% in acetonitrile and added to the column at a flow rate of 1.5-2 column volumes per hour. The column is washed with 2 column volumes of the acetonitrile-glycine buffer. The pGRF precursor is eluted by a gradient formed by three column volumes of 10% acetonitrile-0.02 M glycine mixed with three column volumes 50% acetonitrile-0.02 M glycine. Fractions of 0.1 column volume are collected and assayed for the pGRF precursor.

The pGRF precursor-containing material is then chromatographed over a Sephadex® G15 column equilibrated in 0.25 M acetic acid. The O.D.$_{276}$ peak is then isolated and lyophilized until further use.

**Claims**

1. A DNA compound which comprises an isolated DNA sequence encoding a polypeptide compound of the formula

```
A-Tyr-Ala-Asp-Ala-Ile-Phe-Thr-Asn-B-Tyr-Arg-Lys-Val-
Leu-Gly-Gln-Leu-Ser-Ala-Arg-Lys-Leu-Leu-Gln-Asp-Ile-
Met-Ser-Arg-Gln-Gln-Gly-Glu-Arg-Asn-Gln-Glu-Gln-Gly-
Ala-Arg-Val-Arg-Leu-Gly-Arg-Gln-Val-Asp-Ser-Met-Trp-
Ala-Asp-Gln-Lys-Gln-Met-Ala-Leu-Glu-Ser-Ile-Leu-Ala-
Thr-Leu-Leu-Gln-Glu-His-Arg-Asn-Ser-Gln-Gly-OH;
```

```
wherein A is hydrogen or H-Met-Leu-Leu-Trp-Val-Phe-
Phe-Leu-Val-Thr-Leu-Thr-Leu-Ser-Ser-Gly-Ser-Leu-Ser-
Gln-Pro-Leu-Arg-Met-Pro-Arg and B is Asn or Ser.
```

2. A DNA compound of Claim 1 which comprises an isolated DNA sequence encoding a polypeptide compound of the formula

```
H-Tyr-Ala-Asp-Ala-Ile-Phe-Thr-Asn-Ser-Tyr-Arg-Lys-Val-
Leu-Gly-Gln-Leu-Ser-Ala-Arg-Lys-Leu-Leu-Gln-Asp-Ile-
Met-Ser-Arg-Gln-Gln-Gly-Glu-Arg-Asn-Gln-Glu-Gln-Gly-
Ala-Arg-Val-Arg-Leu-Gly-Arg-Gln-Val-Asp-Ser-Met-Trp-
Ala-Asp-Gln-Lys-Gln-Met-Ala-Leu-Glu-Ser-Ile-Leu-Ala-
Thr-Leu-Leu-Gln-Glu-His-Arg-Asn-Ser-Gln-Gly-OH.
```

3. A DNA compound of Claim 1 which comprises an isolated DNA sequence encoding a polypeptide compound of the formula

```
H-Tyr-Ala-Asp-Ala-Ile-Phe-Thr-Asn-Asn-Tyr-Arg-Lys-Val-
Leu-Gly-Gln-Leu-Ser-Ala-Arg-Lys-Leu-Leu-Gln-Asp-Ile-
Met-Ser-Arg-Gln-Gln-Gly-Glu-Arg-Asn-Gln-Glu-Gln-Gly-
Ala-Arg-Val-Arg-Leu-Gly-Arg-Gln-Val-Asp-Ser-Met-Trp-
Ala-Asp-Gln-Lys-Gln-Met-Ala-Leu-Glu-Ser-Ile-Leu-Ala-
Thr-Leu-Leu-Gln-Glu-His-Arg-Asn-Ser-Gln-Gly-OH.
```

4. The isolated DNA sequence of Claim 3 which is

17

```
TATGCAGATGCCATCTTCACCAACAACTACCGGAAGGTGCTGGGCCA
---------+---------+--------+---------+-------
ATACGTCTACGGTAGAAGTGGTTGTTGATGGCCTTCCACGACCCGGT

GCTCTCTGCCCGAAAGCTCCTCCAGGACATCATGAGCAGGCAGCAGG
--+---------+---------+---------+---------+----
CGAGAGACGGGCTTTCGAGGAGGTCCTGTAGTACTCGTCCGTCGTCC

GGGAGAGAAACCAGGAGCAAGGAGCAAGGGTACGGCTTGGCCGTCAG
-----+---------+---------+---------+---------+-
CCCTCTCTTTGGTCCTCGTTCCTCGTTCCCATGCCGAACCGGCAGTC

GTGGACAGCATGTGGGCAGACCAAAAGCAGATGGCATTGGAGAGCAT
--------+---------+---------+---------+---------
CACCTGTCGTACACCCGTCTGGTTTTCGTCTACCGTAACCTCTCGTA

CCTGGCGACCCTGCTGCAGGAGCACAGGAATTCCCAAGGATGA
---------+---------+---------+---------+---
GGACCGCTGGGACGACGTCCTCGTGTCCTTAAGGGTTCCTACT    .
```

5.  The isolated DNA sequence of Claim 2 which is

```
TATGCAGATGCCATCTTCACCAACAGCTACCGGAAGGTGCTGGGCCA
---------+---------+---------+---------+-------
ATACGTCTACGGTAGAAGTGGTTGTCGATGGCCTTCCACGACCCGGT

GCTCTCTGCCCGAAAGCTCCTCCAGGACATCATGAGCAGGCAGCAGG
--+---------+---------+---------+---------+----
CGAGAGACGGGCTTTCGAGGAGGTCCTGTAGTACTCGTCCGTCGTCC

GGGAGAGAAACCAGGAGCAAGGAGCAAGGGTACGGCTTGGCCGTCAG
-----+---------+---------+---------+---------+-
CCCTCTCTTTGGTCCTCGTTCCTCGTTCCCATGCCGAACCGGCAGTC

GTGGACAGCATGTGGGCAGACCAAAAGCAGATGGCATTGGAGAGCAT
--------+---------+---------+---------+---------
CACCTGTCGTACACCCGTCTGGTTTTCGTCTACCGTAACCTCTCGTA

CCTGGCGACCCTGCTGCAGGAGCACAGGAATTCCCAAGGATGA
---------+---------+---------+---------+---
GGACCGCTGGGACGACGTCCTCGTGTCCTTAAGGGTTCCTACT    .
```

6.  A DNA compound of Claim 1 which comprises an isolated DNA sequence encoding a polypeptide compound of the formula

```
H-Met-Leu-Leu-Trp-Val-Phe-Phe-Leu-Val-Thr-Leu-Thr-Leu-
  Ser-Ser-Gly-Ser-Leu-Ser-Ser-Leu-Pro-Ser-Gln-Pro-Leu-
  Arg-Met-Pro-Arg-Tyr-Ala-Asp-Ala-Ile-Phe-Thr-Asn-Asn-
  Tyr-Arg-Lys-Val-Leu-Gly-Gln-Leu-Ser-Ala-Arg-Lys-Leu-
  Leu-Gln-Asp-Ile-Met-Ser-Arg-Gln-Gln-Gly-Glu-Arg-Asn-
  Gln-Glu-Gln-Gly-Ala-Arg-Val-Arg-Leu-Gly-Arg-Gln-Val-
  Asp-Ser-Met-Trp-Ala-Asp-Gln-Lys-Gln-Met-Ala-Leu-Glu-
  Ser-Ile-Leu-Ala-Thr-Leu-Leu-Gln-Glu-His-Arg-Asn-Ser-
  Gln-Gly-OH.
```

7.  A DNA compound of Claim 1 which comprises an isolated DNA sequence encoding a polypeptide compound of the formula

```
H-Met-Leu-Leu-Trp-Val-Phe-Phe-Leu-Val-Thr-Leu-Thr-Leu-
  Ser-Ser-Gly-Ser-Leu-Ser-Ser-Leu-Pro-Ser-Gln-Pro-Leu-
  Arg-Met-Pro-Arg-Tyr-Ala-Asp-Ala-Ile-Phe-Thr-Asn-Ser-
  Tyr-Arg-Lys-Val-Leu-Gly-Gln-Leu-Ser-Ala-Arg-Lys-Leu-
  Leu-Gln-Asp-Ile-Met-Ser-Arg-Gln-Gln-Gly-Glu-Arg-Asn-
  Gln-Glu-Gln-Gly-Ala-Arg-Val-Arg-Leu-Gly-Arg-Gln-Val-
  Asp-Ser-Met-Trp-Ala-Asp-Gln-Lys-Gln-Met-Ala-Leu-Glu-
  Ser-Ile-Leu-Ala-Thr-Leu-Leu-Gln-Glu-His-Arg-Asn-Ser-
  Gln-Gly-OH.
```

8.  The isolated DNA sequence of Claim 6 which is

```
                          ATGCTGCTCTGGGTGTTCTTCCTCGTCACCCT
                          ---------+---------+---------+--
                          TACGACGAGACCCACAAGAAGGAGCAGTGGGA

      CACCCTCAGCAGCGGCTCCCTCAGCTCCCTGCCCTCCCAGCCCCTCAGGATGCCGC
      -------+---------+---------+---------+---------+------+--
      GTGGGAGTCGTCGCCGAGGGAGTCGAGGGACGGGAGGGTCGGGGAGTCCTACGGCG

      GGTATGCAGATGCCATCTTCACCAACAACTACCGGAAGGTGCTGGGCCAGCTCTCT
      -------+---------+---------+---------+---------+--------
      CCATACGTCTACGGTAGAAGTGGTTGTTGATGGCCTTCCACGACCCGGTCGAGAGA

      GCCCGAAAGCTCCTCCAGGACATCATGAGCAGGCAGCAGGGGGAGAGAAACCAGGA
      -+---------+---------+---------+---------+---------+----
      CGGGCTTTCGAGGAGGTCCTGTAGTACTCGTCCGTCGTCCCCCTCTCTTTGGTCCT

      GCAAGGAGCAAGGGTACGGCTTGGCCGTCAGGTGGACAGCATGTGGGCAGACCAAA
      -----+---------+---------+---------+---------+---------+
      CGTTCCTCGTTCCCATGCCGAACCGGCAGTCCACCTGTCGTACACCCGTCTGGTTT

      AGCAGATGGCATTGGAGAGCATCCTGGCGACCCTGCTGCAGGAGCACAGGAATTCC
      ---------+---------+---------+---------+---------+------
      TCGTCTACCGTAACCTCTCGTAGGACCGCTGGGACGACGTCCTCGTGTCCTTAAGG

      CAAGGATGA
      ---+-----
      GTTCCTACT   .
```

9. The isolated DNA sequence of Claim 7 which is

```
                          ATGCTGCTCTGGGTGTTCTTCCTCGTCACCCT
                          ---------+---------+---------+--
                          TACGACGAGACCCACAAGAAGGAGCAGTGGGA

      CACCCTCAGCAGCGGCTCCCTCAGCTCCCTGCCCTCCCAGCCCCTCAGGATGCCGC
      -------+---------+---------+---------+---------+--------
      GTGGGAGTCGTCGCCGAGGGAGTCGAGGGACGGGAGGGTCGGGGAGTCCTACGGCG

      GGTATGCAGATGCCATCTTCACCAGCAACTACCGGAAGGTGCTGGGCCAGCTCTCT
      --+---------+---------+---------+---------+---------+--
      CCATACGTCTACGGTAGAAGTGGTCGTTGATGGCCTTCCACGACCCGGTCGAGAGA

      GCCCGAAAGCTCCTCCAGGACATCATGAGCAGGCAGCAGGGGGAGAGAAACCAGGA
      -------+---------+---------+---------+---------+--------
      CGGGCTTTCGAGGAGGTCCTGTAGTACTCGTCCGTCGTCCCCCTCTCTTTGGTCCT

      GCAAGGAGCAAGGGTACGGCTTGGCCGTCAGGTGGACAGCATGTGGGCAGACCAAA
      --+---------+---------+---------+---------+---------+---
      CGTTCCTCGTTCCCATGCCGAACCGGCAGTCCACCTGTCGTACACCCGTCTGGTTT

      AGCAGATGGCATTGGAGAGCATCCTGGCGACCCTGCTGCAGGAGCACAGGAATTCC
      ------+---------+---------+---------+---------+---------
      TCGTCTACCGTAACCTCTCGTAGGACCGCTGGGACGACGTCCTCGTGTCCTTAAGG

      CAAGGATGA
      +--------
      GTTCCTACT   .
```

20

10. A DNA compound which comprises an isolated DNA sequence which is

```
TATGCAGATGCCATCTTCACCAACAACTACCGGAAGGTGCTGGGCCA
----------+----------+----------+----------+-------
ATACGTCTACGGTAGAAGTGGTTGTTGATGGCCTTCCACGACCCGGT

GCTCTCTGCCCGAAAGCTCCTCCAGGACATCATGAGCAGGCAGCAGG
--+----------+----------+----------+----------+----
CGAGAGACGGGCTTTCGAGGAGGTCCTGTAGTACTCGTCCGTCGTCC

GGGAGAGAAACCAGGAGCAAGGA
-----+----------+-------
CCCTCTCTTTGGTCCTCGTTCCT    .
```

11. A DNA compound which comprises an isolated DNA sequence which is

```
TATGCAGATGCCATCTTCACCAACAGCTACCGGAAGGTGCTGGGCCA
----------+----------+----------+----------+-------
ATACGTCTACGGTAGAAGTGGTTGACGATGGCCTTCCACGACCCGGT

GCTCTCTGCCCGAAAGCTCCTCCAGGACATCATGAGCAGGCAGCAGG
--+----------+----------+----------+----------+----
CGAGAGACGGGCTTTCGAGGAGGTCCTGTAGTACTCGTCCGTCGTCC

GGGAGAGAAACCAGGAGCAAGGA
-----+----------+-------
CCCTCTCTTTGGTCCTCGTTCCT    .
```

12. A polypeptide compound of the formula

```
A-Tyr-Ala-Asp-Ala-Ile-Phe-Thr-Asn-B-Tyr-Arg-Lys-Val-
Leu-Gly-Gln-Leu-Ser-Ala-Arg-Lys-Leu-Leu-Gln-Asp-Ile-
Met-Ser-Arg-Gln-Gln-Gly-Glu-Arg-Asn-Gln-Glu-Gln-Gly-
Ala-Arg-Val-Arg-Leu-Gly-Arg-Gln-Val-Asp-Ser-Met-Trp-
Ala-Asp-Gln-Lys-Gln-Met-Ala-Leu-Glu-Ser-Ile-Leu-Ala-
Thr-Leu-Leu-Gln-Glu-His-Arg-Asn-Ser-Gln-Gly-OH
```

```
wherein A is hydrogen or H-Met-Leu-Leu-Trp-Val-Phe-
Phe-Leu-Val-Thr-Leu-Thr-Leu-Ser-Ser-Gly-Ser-Leu-Ser-
Gln-Pro-Leu-Arg-Met-Pro-Arg; B is Asn or Ser; and
the pharmaceutically acceptable acid or carboxylic
acid addition salts thereof.
```

the pharmaceutically acceptable acid or carboxylic acid addition salts thereof.

13. The polypeptide compound of Claim 12 that is

H-Tyr-Ala-Asp-Ala-Ile-Phe-Thr-Asn-Asn-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Arg-Asn-Gln-Glu-Gln-Gly-Ala-Arg-Val-Arg-Leu-Gly-Arg-Gln-Val-Asp-Ser-Met-Trp-Ala-Asp-Gln-Lys-Gln-Met-Ala-Leu-Glu-Ser-Ile-Leu-Ala-Thr-Leu-Leu-Gln-Glu-His-Arg-Asn-Ser-Gln-Gly-OH; and

the pharmaceutically acceptable acid or carboxylic acid addition salts thereof.

14. The polypeptide compound of Claim 12 that is

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Arg-Asn-Gln-Glu-Gln-Gly-Ala-Arg-Val-Arg-Leu-Gly-Arg-Gln-Val-Asp-Ser-Met-Trp-Ala-Asp-Gln-Lys-Gln-Met-Ala-Leu-Glu-Ser-Ile-Leu-Ala-Thr-Leu-Leu-Gln-Glu-His-Arg-Asn-Ser-Gln-Gly-OH; and

the pharmaceutically acceptable acid or carboxylic acid addition salts thereof.

15. The polypeptide compound of Claim 12 that is

H-Met-Leu-Leu-Trp-Val-Phe-Phe-Leu-Val-Thr-Leu-Thr-Leu-Ser-Ser-Gly-Ser-Leu-Ser-Ser-Leu-Pro-Ser-Gln-Pro-Leu-Arg-Met-Pro-Arg-Tyr-Ala-Asp-Ala-Ile-Phe-Thr-Asn-Asn-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Arg-Asn-Gln-Glu-Gln-Gly-Ala-Arg-Val-Arg-Leu-Gly-Arg-Gln-Val-Asp-Ser-Met-Trp-Ala-Asp-Gln-Lys-Gln-Met-Ala-Leu-Glu-Ser-Ile-Leu-Ala-Thr-Leu-Leu-Gln-Glu-His-Arg-Asn-Ser-Gln-Gly-OH; and the pharmaceutically acceptable acid or carboxylic acid addition salts thereof.

16. A method for inducing growth hormone release in an animal which comprises administering an effective amount of a polypeptide compound which is

H-Tyr-Ala-Asp-Ala-Ile-Phe-Thr-Asn-A-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Arg-Asn-Gln-Glu-Gln-Gly-Ala-Arg-Val-Arg-Leu-Gly-Arg-Gln-Val-Asp-Ser-Met-Trp-Ala-Asp-Gln-Lys-Gln-Met-Ala-Leu-Glu-Ser-Ile-Leu-Ala-Thr-Leu-Leu-Gln-Glu-His-Arg-Asn-Ser-Gln-Gly-OH

wherein A is Asn or Ser; and pharmaceutically acceptable acid or carboxylic acid addition salts thereof.

**17.** The method of Claim 16 wherein A is Asn.

**18.** The method of Claim 16 wherein A is Ser.

**19.** A polypeptide compound of the formula

```
H-Tyr-Ala-Asp-Ala-Ile-Phe-Thr-Asn-Asn-Tyr-Arg-Lys-Val-
Leu-Gly-Gln-Leu-Ser-Ala-Arg-Lys-Leu-Leu-Gln-Asp-Ile-
Met-Ser-Arg-Gln-Gln-Gly-Glu-Arg-Asn-Gln-Glu-Gln-Gly-
Ala-Arg-Val-Arg-Leu-A; wherein A is OH or NH2.
```

**Claims for the following Contracting States: GR and ES**

**1.** A method for inducing growth hormone release in an animal which comprises administering an effective amount of a polypeptide compound which is

```
H-Tyr-Ala-Asp-Ala-Ile-Phe-Thr-Asn-A-Tyr-Arg-Lys-Val-
Leu-Gly-Gln-Leu-Ser-Ala-Arg-Lys-Leu-Leu-Gln-Asp-Ile-
Met-Ser-Arg-Gln-Gln-Gly-Glu-Arg-Asn-Gln-Glu-Gln-Gly-
Ala-Arg-Val-Arg-Leu-Gly-Arg-Gln-Val-Asp-Ser-Met-Trp-
Ala-Asp-Gln-Lys-Gln-Met-Ala-Leu-Glu-Ser-Ile-Leu-Ala-
Thr-Leu-Leu-Gln-Glu-His-Arg-Asn-Ser-Gln-Gly-OH
```

wherein A is Asn or Ser; and the pharmaceutically acceptable acid or carboxylic acid addition salts thereof.

**2.** The method of Claim 1 wherein A is Asn.

**3.** The method of Claim 1 wherein A is Ser.

EP 0 459 747 A1

European Patent Office

# PARTIAL EUROPEAN SEARCH REPORT

which under Rule 45 of the European Patent Convention shall be considered, for the purposes of subsequent proceedings, as the European search report

Application number

## DOCUMENTS CONSIDERED TO BE RELEVANT

EP 91304796.5

| Category | Citation of document with indication, where appropriate, of relevant passages | Relevant to claim | CLASSIFICATION OF THE APPLICATION (Int. Cl.5) |
|---|---|---|---|
| Y | EP - B1 - 0 108 387<br>(F. HOFFMANN-LA ROCHE AG)<br>* Claims * | 1-11 | C 12 N 15/16<br>C 07 K 7/10<br>A 61 K 37/43 |
| Y | EP - A1 - 0 212 531<br>(SYNTEX (U.S.A.) INC.)<br>* Claims 1-3,10-14 * | 12-15, 19 | |
| A | US - A - 4 610 976<br>(BOHLEN)<br>* Totality * | 1-15, 19 | |

TECHNICAL FIELDS SEARCHED (Int. Cl.5)

C 12 N
C 07 K
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-15,19
Claims searched incompletely: –
Claims not searched:
Reason for the limitation of the search: 16-18 (Article 52(4) EPC)

Method for treatment of the human or animal body by therapy

| Place of search | Date of completion of the search | Examiner |
|---|---|---|
| VIENNA | 26-08-1991 | AUGUSTIN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1505.1 03.82

24

SEQUENCE LISTING

(1) GENERAL INFORMATION:

    (i) APPLICANT:Eli Lilly and Company

    (ii) TITLE OF INVENTION:PRECURSOR FORMS OF PORCINE GROWTH HORMONE RELEASING FACTOR AND RELATED DNA COMPOUNDS

    (iii) NUMBER OF SEQUENCES: 10

    (iv) CORRESPONDENCE ADDRESS:

        (A) ADDRESSEE:Mr. C. Mark Hudson

        (B) STREET:Erl Wood Manor

        (C) CITY:Windlesham

        (D) STATE:Surrey GU20 6PH

        (E) COUNTRY:Grande Bretagne

        (F) ZIP:

    (v) COMPUTER READABLE FORM:

        (A) MEDIUM TYPE:Diskette, 3.50 inch, 1.0 Mb storage

        (B) COMPUTER:Macintosh

        (C) OPERATING SYSTEM:Macintosh

        (D) SOFTWARE:Microsoft Word

    (vi) CURRENT APPLICATION DATA:

        (A) APPLICATION NUMBER: 91304796.5

        (B) FILING DATE:  28 MAY 1991

        (C) CLASSIFICATION:

    (vii) ATTORNEY/AGENT INFORMATION:

        (A) NAME:Mr. C. Mark Hudson

        (B) REGISTRATION NUMBER:307

(C) REFERENCE/DOCKET NUMBER: X-8172

(ix) TELECOMMUNICATION INFORMATION:

    (A) TELEPHONE:0276 78441

    (B) TELEFAX:0276 78306

    (C) TELEX:858177


(2) INFORMATION FOR SEQ ID NO: 1:

    (i) SEQUENCE CHARACTERISTICS:

        (A) LENGTH: 76 AMINO ACIDS

        (B) TYPE:    PROTEIN

        (C) STRANDEDNESS:  SINGLE

        (D) TOPOLOGY:   LINEAR

   (ii) MOLECULE TYPE:   PROTEIN


   (xi) SEQUENCE DESCRIPTION: SEQ ID NO:1:

```
Tyr Ala Asp Ala Ile Phe Thr Asn Ser Tyr Arg Lys Val Leu Gly
                  5                  10                   15
Gln Leu Ser Ala Arg Lys Leu Leu Gln Asp Ile Met Ser Arg Gln
                 20                  25                   30
Gln Gly Glu Arg Asn Gln Glu Gln Gly Ala Arg Val Arg Leu Gly
                 35                  40                   45
Arg Gln Val Asp Ser Met Trp Ala Asp Gln Lys Gln Met Ala Leu
                 50                  55                   60
Glu Ser Ile Leu Ala Thr Leu Leu Gln Glu His Arg Asn Ser Gln
                 65                  70                   75
Gly
```

(3) INFORMATION FOR SEQ ID NO: 2:

    (i) SEQUENCE CHARACTERISTICS:

        (A) LENGTH: 76 AMINO ACIDS

        (B) TYPE:    PROTEIN

        (C) STRANDEDNESS:  SINGLE

        (D) TOPOLOGY:   LINEAR

   (ii) MOLECULE TYPE:   PROTEIN


   (xi) SEQUENCE DESCRIPTION: SEQ ID NO:2:

```
Tyr Ala Asp Ala Ile Phe Thr Asn Asn Tyr Arg Lys Val Leu Gly
                  5                   10                      15
Gln Leu Ser Ala Arg Lys Leu Leu Gln Asp Ile Met Ser Arg Gln
                 20                   25                      30
Gln Gly Glu Arg Asn Gln Glu Gln Gly Ala Arg Val Arg Leu Gly
                 35                   40                      45
Arg Gln Val Asp Ser Met Trp Ala Asp Gln Lys Gln Met Ala Leu
                 50                   55                      60
Glu Ser Ile Leu Ala Thr Leu Leu Gln Glu His Arg Asn Ser Gln
                 65                   70                      75
Gly
```

(4) INFORMATION FOR SEQ ID NO: 3:

  (i) SEQUENCE CHARACTERISTICS:

    (A) LENGTH: 106 AMINO ACIDS

    (B) TYPE:    PROTEIN

    (C) STRANDEDNESS:  SINGLE

    (D) TOPOLOGY:    LINEAR

  (ii) MOLECULE TYPE:    PROTEIN


  (xi) SEQUENCE DESCRIPTION: SEQ ID NO:3:
```
Met Leu Leu Trp Val Phe Phe Leu Val Thr Leu Thr Leu Ser Ser
                  5                   10                      15
Gly Ser Leu Ser Ser Leu Pro Ser Gln Pro Leu Arg Met Pro Arg
                 20                   25                      30
Tyr Ala Asp Ala Ile Phe Thr Asn Ser Tyr Arg Lys Val Leu Gly
                 35                   40                      45
Gln Leu Ser Ala Arg Lys Leu Leu Gln Asp Ile Met Ser Arg Gln
                 50                   55                      60
Gln Gly Glu Arg Asn Gln Glu Gln Gly Ala Arg Val Arg Leu Gly
                 65                   70                      75
Arg Gln Val Asp Ser Met Trp Ala Asp Gln Lys Gln Met Ala Leu
                 80                   85                      90
Glu Ser Ile Leu Ala Thr Leu Leu Gln Glu His Arg Asn Ser Gln
                 95                  100                     105
Gly
```

(5) INFORMATION FOR SEQ ID NO: 4:

  (i) SEQUENCE CHARACTERISTICS:

    (A) LENGTH: 106 AMINO ACIDS

    (B) TYPE:    PROTEIN

    (C) STRANDEDNESS:  SINGLE

    (D) TOPOLOGY:    LINEAR

(ii) MOLECULE TYPE:    PROTEIN


(xi) SEQUENCE DESCRIPTION: SEQ ID NO:4:
```
Met Leu Leu Trp Val Phe Phe Leu Val Thr Leu Thr Leu Ser Ser
                  5                  10                  15
Gly Ser Leu Ser Ser Leu Pro Ser Gln Pro Leu Arg Met Pro Arg
                 20                  25                  30
Tyr Ala Asp Ala Ile Phe Thr Asn Asn Tyr Arg Lys Val Leu Gly
                 35                  40                  45
Gln Leu Ser Ala Arg Lys Leu Leu Gln Asp Ile Met Ser Arg Gln
                 50                  55                  60
Gln Gly Glu Arg Asn Gln Glu Gln Gly Ala Arg Val Arg Leu Gly
                 65                  70                  75
Arg Gln Val Asp Ser Met Trp Ala Asp Gln Lys Gln Met Ala Leu
                 80                  85                  90
Glu Ser Ile Leu Ala Thr Leu Leu Gln Glu His Arg Asn Ser Gln
                 95                 100                 105
Gly
```

(6) INFORMATION FOR SEQ ID NO: 5:

    (i) SEQUENCE CHARACTERISTICS:

        (A) LENGTH: 231 nucleotides

        (B) TYPE:    DNA

        (C) STRANDEDNESS:   double

        (D) TOPOLOGY:    LINEAR

    (ii) MOLECULE TYPE:    nucleic acid


    (xi) SEQUENCE DESCRIPTION: SEQ ID NO:5:
```
TAT GCA GAT GCC ATC TTC ACC AAC AAC TAC 30
CGG AAG GTG CTG GGC CAG CTC TCT GCC CGA 60
AAG CTC CTC CAG GAC ATC ATG AGC AGG CAG 90
CAG GGG GAG AGA AAC CAG GAG CAA GGA GCA 120
AGG GTA CGG CTT GGC CGT CAG GTG GAC AGC 150
ATG TGG GCA GAC CAA AAG CAG ATG GCA TTG 180
GAG AGC ATC CTG GCG ACC CTG CTG CAG GAG 210
CAC AGG AAT TCC CAA GGA TGA 231
```
(7) INFORMATION FOR SEQ ID NO: 6:

    (i) SEQUENCE CHARACTERISTICS:

        (A) LENGTH: 231 nucleotides

        (B) TYPE:    DNA

        (C) STRANDEDNESS:   double

(D) TOPOLOGY:    LINEAR

(ii) MOLECULE TYPE:    nucleic acid

(xi) SEQUENCE DESCRIPTION: SEQ ID NO:6:
```
TAT GCA GAT GCC ATC TTC ACC AAC AGC TAC 30
CGG AAG GTG CTG GGC CAG CTC TCT GCC CGA 60
AAG CTC CTC CAG GAC ATC ATG AGC AGG CAG 90
CAG GGG GAG AGA AAC CAG GAG CAA GGA GCA 120
AGG GTA CGG CTT GGC CGT CAG GTG GAC AGC 150
ATG TGG GCA GAC CAA AAG CAG ATG GCA TTG 180
GAG AGC ATC CTG GCG ACC CTG CTG CAG GAG 210
CAC AGG AAT TCC CAA GGA TGA 231
```

(8)  INFORMATION FOR SEQ ID NO: 7:

(i) SEQUENCE CHARACTERISTICS:

(A)  LENGTH: 321 nucleotides

(B)  TYPE:    DNA

(C)  STRANDEDNESS:  double

(D)  TOPOLOGY:    LINEAR

(ii) MOLECULE TYPE:    nucleic acid

(xi) SEQUENCE DESCRIPTION: SEQ ID NO:7:
```
ATG CTG CTC TGG GTG TTC TTC CTC GTC ACC 30
CTC ACC CTC AGC AGC GGC TCC CTC AGC TCC 60
CTG CCC TCC CAG CCC CTC AGG ATG CCG CGG 90
TAT GCA GAT GCC ATC TTC ACC AAC AGC TAC 120
CGG AAG GTG CTG GGC CAG CTC TCT GCC CGA 150
AAG CTC CTC CAG GAC ATC ATG AGC AGG CAG 180
CAG GGG GAG AGA AAC CAG GAG CAA GGA GCA 210
AGG GTA CGG CTT GGC CGT CAG GTG GAC AGC 240
ATG TGG GCA GAC CAA AAG CAG ATG GCA TTG 270
GAG AGC ATC CTG GCG ACC CTG CTG CAG GAG 300
CAC AGG AAT TCC CAA GGA TGA 321
```

(9)  INFORMATION FOR SEQ ID NO: 8:

(i) SEQUENCE CHARACTERISTICS:

(A)  LENGTH: 321 nucleotides

(B)  TYPE:    DNA

(C)  STRANDEDNESS:  double

(D)  TOPOLOGY:    LINEAR

(ii) MOLECULE TYPE:    nucleic acid

(xi) SEQUENCE DESCRIPTION: SEQ ID NO:8:
```
ATG CTG CTC TGG GTG TTC TTC CTC GTC ACC 30
CTC ACC CTC AGC AGC GGC TCC CTC AGC TCC 60
CTG CCC TCC CAG CCC CTC AGG ATG CCG CGG 90
TAT GCA GAT GCC ATC TTC ACC AAC AAC TAC 120
CGG AAG GTG CTG GGC CAG CTC TCT GCC CGA 150
AAG CTC CTC CAG GAC ATC ATG AGC AGG CAG 180
CAG GGG GAG AGA AAC CAG GAG CAA GGA GCA 210
AGG GTA CGG CTT GGC CGT CAG GTG GAC AGC 240
ATG TGG GCA GAC CAA AAG CAG ATG GCA TTG 270
GAG AGC ATC CTG GCG ACC CTG CTG CAG GAG 300
CAC AGG AAT TCC CAA GGA TGA 321
```

(10) INFORMATION FOR SEQ ID NO: 9:

(i) SEQUENCE CHARACTERISTICS:

(A) LENGTH: 117 nucleotides

(B) TYPE:    DNA

(C) STRANDEDNESS:   double

(D) TOPOLOGY:    LINEAR

(ii) MOLECULE TYPE:    nucleic acid

(xi) SEQUENCE DESCRIPTION: SEQ ID NO:9:
```
TAT GCA GAT GCC ATC TTC ACC AAC AAC TAC 30
CGG AAG GTG CTG GGC CAG CTC TCT GCC CGA 60
AAG CTC CTC CAG GAC ATC ATG AGC AGG CAG 90
CAG GGG GAG AGA AAC CAG GAG CAA GGA 117
```
(11) INFORMATION FOR SEQ ID NO: 10:

(i) SEQUENCE CHARACTERISTICS:

(A) LENGTH: 117 nucleotides

(B) TYPE:    DNA

(C) STRANDEDNESS:   double

(D) TOPOLOGY:    LINEAR

(ii) MOLECULE TYPE:    nucleic acid

(xi) SEQUENCE DESCRIPTION: SEQ ID NO:10:
```
TAT GCA GAT GCC ATC TTC ACC AAC AGC TAC 30
```

EP 0 459 747 A1

```
CGG AAG GTG CTG GGC CAG CTC TCT GCC CGA  60
AAG CTC CTC CAG GAC ATC ATG AGC AGG CAG  90
CAG GGG GAG AGA AAC CAG GAG CAA GGA      117
```